Europäisches Patentamt

European Patent Office

Office européen des brevets

(11) Publication number: **0 308 181**
**A1**

# (12) EUROPEAN PATENT APPLICATION

(21) Application number: **88308460.0**

(22) Date of filing: **13.09.88**

(51) Int. Cl.⁴: **A 61 K 47/00**
**A 61 K 37/24**

(30) Priority: **14.09.87 DK 4774/87**
**23.12.87 DK 6825/87**

(43) Date of publication of application:
**22.03.89 Bulletin 89/12**

(84) Designated Contracting States:
**AT BE CH DE ES FR GB GR IT LI LU NL SE**

(71) Applicant: **NOVO INDUSTRI A/S**
**Novo Allé**
**DK-2880 Bagsvaerd (DK)**

(72) Inventor: **Sörensen, Anders Robert**
**26, Sennepshaven**
**DK-2730 Herlev (DK)**

**Engesgaard, Anne**
**31 Padborgvej**
**DK-2610 Rödovre (DK)**

**Hansen, Philip Edgar**
**29, Teglstrupvej**
**DK-2100 Köbenhavn (DK)**

(74) Representative: **Brown, John David et al**
**FORRESTER & BOEHMERT Widenmayerstrasse 4/I**
**D-8000 München 22 (DE)**

(54) Trans-mucosal delivery formulations and a method for preparation thereof.

(57) The systemic absorption after non-enteral, transmucosal delivery of certain drugs, in particular pharmacologically active polypeptides, is enhanced from formulations containing a monosaccharide or an oligosaccharide, preferably a cyclodextrin.

EP 0 308 181 A1

## Description

## TRANS-MUCOSAL DELIVERY FORMULATIONS AND A METHOD FOR PREPARATION THEREOF

The present invention is in the field of drug delivery by non-enteral, trans-mucosal routes of administration. In particular, the present invention relates to novel pharmaceutical preparations adapted for oral mucosal, rectal or nasal administration and to a method for preparing such preparations.

## BACKGROUND OF THE INVENTION

While non-invasive medication, such as oral, oral mucosal or rectal administration of a drug is undoubtedly most convenient to the patient, parenteral drug delivery is usually regarded as being the most effective. In particular, drugs which are inactivated in or poorly absorbed by the gastrointestinal tract and drugs which are subject to extensive first passage hepatic metabolism following oral administration are usually administered parenterally.

There are obvious inconveniences associated with parenteral drug administration, such as the need for sterile delivery devices, pain, irritation and eventually possible tissue damage caused by reiterated injections and a potential risk of infection. Therefore, alternative means of drug delivery, equalling parenteral administration in the sense that first passage hepatic metabolism is circumvented, have been sought. Examples of such potentially promising alternatives are drug administration via oral mucosal, rectal or nasal routes. However, it is a general experience with these methods for non-invasive medication that the bioavailability of the drug is largely unpredictable, being highly dependent on such factors as the actual choice of route of delivery, the chemical nature of the drug and on the selection of suitable absorption promoting adjuvants or vehicles.

Rectal administration of a variety of drugs, e.g. as suppositories, is of long standing in medical practice. Administration via the rectal route permits the drug to enter the circulation directly without first passing the liver. This was shown for propranolol by A.G. de Boer et al. (J.Pharm.Pharmacol. 33 (1981), 50). Propranolol is also absorbed from the nasal cavity. The blood level attained is almost equal to that of intravenous administration as is also the case with intranasal delivery of progesterone.

Other examples of intranasal formulations of pharmaceutically active agents with molecular weights up to about 1 kD are known, for example compositions containing ergopeptide alkaloids dissolved in aqueous ethanol administered as aerosols (Swiss Patent No. 636,011), salts of pharmaceutically active amines with fatty acids (Canadian Patent No. 988,852) and catecholamine suspended in a fatty acid (or ester) emulsified with polyoxyethylene (European Patent Publication No. 0 160 501 A).

Over the last decades a variety of (mainly synthetic) polypetide drugs have been developed. In general, polypeptides have been administered parenterally due to incomplete absorption from and digestive instability in the alimentary canal. This is probably the reason why in particular studies of the trans-mucosal and particularly of nasal delivery of polypeptides have been intensified during recent years. It has been found that while some smaller polypeptides (up to about 10 amino acid residues) may be reasonably well absorbed intranasally from simple aqueous formulations, generally the nasal bioavailability of larger polypeptides becomes both incomplete and variable, and increasingly so with increasing molecular weight (for review, see L. Illum: Archiv for Pharmaci og Chemi 94 (1987), 127-135).

With a view to overcoming the disadvantages encountered with trans-mucosal, and particularly with nasal delivery compositions containing larger polypeptides, the additional incorporation of a variety of biocompatible absorption promoting agents has been devised, in particular so-called enhancers for liquid formulations and powder vehicles for solid formulations.

In this respect reference is made to European Patent Publication No. 0 111 841 A, disclosing the absorption enhancing effect of a bile acid and to U.S. Patent No. 4,476,116, using chelating agents such as EDTA.

Trans-mucosal formulations adapted to insulin delivery would naturally be highly preferred by the insulin dependent diabetic patient to the presently available preparations for parenteral administration provided that the insulin is absorbed to a reasonably effective and constant extent from the chosen cavity. A variety of absorption enhancing agents, mainly surfactants, have been devised for such formulations.

A modest absorption by oral mucosal delivery of insulin in the presence of sodium glycocholate has been observed in dogs (M. Ishida et al.: Chem.Pharm.Bulletin 29 (1981), 810-816) . Similar results have been obtained by rectal administration, both in the presence of surfactants and of non-surfactants (T. Nishitate et al.: Journ.Pharm.Sciences 69 (1980), 744-745).

Ionic as well as non-ionic surfactant enhancers, such as bile acid salts and polyoxyethylene higher alcohol ethers incorporated into nasal formulations are disclosed in British Patent No. 1,527,605 while the use of a specific polyoxyethylene higher alcohol ether, namely polyoxyethylene-9 lauryl ether is described in: R. Salzman et al., New England J. of Med. 312 (1985), 1078-1084. Other enhancers, for example salts of taurodihydrofusidic acid, are disclosed in U.S. Patent No. 4,548,922.

The chemical structure of these prior art enhancers deviate considerably from those of known constituents of cellular membranes, including those of the nasal cavity. This feature could possibly explain their general proneness to cause nasal irritation or even permanent damage to the nasal membrane, particularly during chronic administration. According to Danish Patent Application No. 3700/87 enhancers more closely related to mucous membrane components, such as medium chain length phospholipids, are considerably more effective and well tolerated. Seemingly acceptable vehicles for nasal powder formulations are such water insoluble bases as cellulose, starch and their chemical derivatives, other polymers, or other macromolecular vehicles disclosed by T. Nagai and co-workers, e.g. in U.S. Patent No. 4,613,500.

The present invention is based on the observation that certain water soluble saccharides exhibit a surprisingly high efficacy as absorption enhancing adjuvants and, furthermore, seem to be well tolerated by chronic administration to mucosal surfaces.

## SUMMARY OF THE INVENTION

Thus, according to its first aspect the present invention provides a preparation for trans-mucosal drug delivery, which preparation comprises a pharmaceutically active agent and an absorption enhancing vehicle comprising at least one saccharide or a metabolite thereof selected from the group consisting of a monosaccharide containing from 3 to 8 carbon atoms and an oligosaccharide containing from 6 to 16 monosaccharide, preferably glucosyl, residues.

According to a second aspect of the present invention a method is provided for making a preparation for trans-mucosal drug delivery, which method comprises dispersing the pharmaceutically active agent in a vehicle, e.g. by mechanical means or by lyophilization of an aqueous solution or suspension, which vehicle comprises at least one saccharide as defined in Claim 1 hereinafter and, optionally, one or more ancillary constituents, such as a powdery, waxy or liquid diluent, and pH-buffering, preserving and osmotic pressure controlling agents.

## PREFERRED EMBODIMENTS OF THE INVENTION AND DETAILED DESCRIPTION THEREOF

Preferred monosaccharides are selected from the group consisting of tetroses, pentoses, hexoses and heptoses. Particularly preferred are D-erythrose, D-ribose, D-ribulose, D-xylose, D-lyxose, L-arabinose, D-mannose, L-sorbose, and D-sedoheptulose, all of which may be found in nature. However, the corresponding enantiomer monosaccharides are also contemplated within the scope of this invention. Most preferred monosaccharides are D-lyxose, D-xylose, D-ribose, D-mannose and L-sorbose. Examples of monosaccharide metabolites are monosaccharide phosphates, in particular those phosphorylated at a terminal hydroxyl group.

A preferred group of oligosaccharides comprises α-, β-, γ-cyclodextrin, O-substituted derivatives thereof, preferably those substituted with acyl, carboxyalkyl or alkyl, more preferable with methyl, and corresponding branched cyclodextrins wherein the branch consists of a carbohydrate residue, in particular a maltosyl or glucosyl residue.

Most preferred oligosaccharides are α-, β-, and γ-cyclodextrin and derivatives thereof carrying one or more maltosyl branches. Branched cyclodextrins are known in the art, e.g. from U.S. Patent No. 4,668,626.

In another preferred embodiment of this invention the pharmaceutically active agent is a polypeptide. One group of preferred polypeptides is insulin and insulin derivatives, e.g. insulin modified by chemical or enzymatic methods or by recombinant DNA technology, or mixtures of such insulins, proinsulin and glucagon. Other preferred polypeptides are parathyroid hormone, parathyroid hormone antagonist, calcitonin, vasopressin, renin, prolactin, growth hormone, thyroid stimulating hormone, corticotropin, corticotropin-releasing factor, follicle stimulating hormone, luteinizing hormone, chorionic gonadotropin, atrial peptides, interferon, tissue plasminogen activator, gammaglobulins, Factor VII, Factor VIII, growth hormone releasing hormone, luteinizing hormone releasing hormone, somatostatin and cholecystokinins.

The preparation of this invention may be solid, e.g. a suppository for rectal administration or a powder acceptable for snuffing, or it may be liquid, e.g. a solution or suspension adapted for administration as a spray.

Powder formulations for nasal use may contain the pharmaceutically active agent and the absorption enhancing vehicle of this invention in admixture with nasally acceptable water-insoluble, powdery diluents or mixtures thereof, e.g. cellulose or derivatives thereof, for example cellulose ethers or sodium carboxymethylcellulose, starch, a long chain fatty acid or a salt thereof, e.g. aluminum or magnesium stearate, an organic polymer, e.g. of acrylic acid or a derivative or salt thereof or inorganic diluents, such as talc or diatomaceous earth. It is realized (c.f. U.S. Patent No. 4,613,500, supra) that these and other similar compounds may themselves function as vehicles, e.g. for intranasal delivery. However, within the context of the present invention, directed to substantially more effective vehicles, the term diluent is used for these prior art vehicles.

In a preferred mode the amount of diluent admixed with the water-soluble vehicle is in the range of from 0 to 80%, more preferred from 0 to 50% by weight. In the most preferred embodiment of this invention the water-soluble vehicle is undiluted.

Powdery preparations may be prepared by dispersing the solid, e.g. crystalline, amorphous or lyophilized pharmaceutically active agent in the powdery vehicle, optionally admixed with a diluent, the dispersion being effected by mechanical means, e.g. in a mortar or by milling. Alternatively, the pharmaceutically active agent may be dissolved or suspended in water together with the vehicle, optionally admixed with the water-insoluble diluent, if necessary followed by adjustment of pH to neutrality, i.e. to pH in the range from about 6.5 to about 8. The suspension may then be taken to dryness, e.g. by lyophilization.

Due to the fact that proteases and peptidases are associated with the nasal mucosa (see R.E. Stratford and V.H.L. Lee: Int.Journ.Pharmaceutics 30 (1986), 73-82) it may be desirable to incorporate biocompatible protease and peptidase inhibitors into polypeptide containing formulations.

Liquid preparations wherein the diluent is water, will usually include ancillary agents, such as a pH-buffering system, e.g. a phosphate, citrate or acetate buffer, a preservative and an osmotic pressure controlling agent, e.g. glycerol or sodium chloride.

Liquid preparations may also combine the concept of the present invention with that of Danish Patent Application No. 3700/87, supra, directed to such absorption enhancers as medium chain phosphatidylcholines and fatty oils. Such liquid preparations could contain the pharmaceutically active agent and the water-soluble saccharide dissolved and/or dispersed in a solution of the phosphatidylcholine(s) in the fatty oil or in an emulsion thereof with an aqueous phase.

The concentration of the pharmaceutically active agent in the preparations of this invention will of course depend on the particular agent chosen, on its efficacy, on a comparison of its bioavailability by trans-mucosal administration and by other routes of administration, for example parenteral injection, and on the desired frequency of administration combined with the desired single dosage of the formulation. Such pharmacological data can routinely be so obtained by the skilled artisan from animal experiments, for example in terms of index values, such as those estimated for insulin preparations in the examples hereinafter provided.

Taking insulin as an example, its concentration in the preparation of this invention may be in the range of from about 5 to 10000 international units (IU) per gram, preferably from 50 to 5000 IU per gram.

Taking glucagon as an example, its concentration in the preparation of this invention may be in the range of from about 0.1 to 800 mg per gram, preferably from 1 to 600 mg per gram.

The insulin preparations of this invention preferably contain bovine, porcine or human insulin.

An exemplary mode of preparing the insulin preparations of this invention comprises dissolving insulin, for example crystalline zinc insulin, for example the highly purified grade of insulin disclosed in British Patent No. 1,285,023, in water in the presence of an acid, for example hydrochloric acid. An aqueous solution of a preservative, for example phenol, an alkyl phenol, such as cresol, or methyl p-hydroxybenzoate, is prepared separately. optionally also containing an agent rendering the solution isotonic, such as sodium chloride or glycerol. Furthermore, the preservative solution may contain a buffering agent, such as sodium phosphate, sodium citrate, sodium acetate or TRIS (tris(hydroxymethyl)aminomethane) and a protease inhibitor. The resulting preservative solution is then admixed with the acid insulin solution followed by addition of a base, for example a sodium hydroxide solution, to adjust the pH value to neutrality. The saccharide vehicle may be added to the insulin solution as an aquous solution or suspension. Alternatively the saccharide solution or suspension may, if desired, contain the buffering agent and preservative. After mixing, the pH value of the insulin preparation may be readjusted to neutrality. Finally, the resulting mixture is de-watered, e.g. by lyophilization.

Powder formulations of this invention may be used in any dosage dispensing device adapted for trans-mucosal administration. As an example, intranasal delivery may be accomplished from a capsule containing the desired dosage of the powder formulation. By insertion in a nasal insufflator provided with an adaptor the capsule is penetrated with a needle, thereby producing a hole in each end. The adaptor is then contacted with the nostril and the capsule emptied either by means of an air stream (e.g. from a mechanical pumping device) or a propellant gas stream.

Liquid preparations may be used in any dosage dispensing device adapted for intranasal administration. The device should be constructed with a view to ascertaining optimum metering accuracy and compatibility of its constructive elements, such as container, valve and actuator with the nasal formulation and could be based on a mechanical pump system, e.g. that of a metered-dose nebulizer, or on a pressurized aerosol system. The aerosol system requires the propellant to be inert towards the formulation. Suitable propellants may be selected among such gases as fluorocarbons, hydrocarbons, nitrogen and dinitrogen oxide or mixtures thereof.

Further details of practising this invention are furnished by way of the following examples which, however, should not be construed so as to impose any kind of limitation to the scope of this invention.

The insulin starting material used in the examples contained about 20 to 30 µg zinc per mg nitrogen.

The monosaccharides and $\alpha$-, $\beta$- and $\gamma$-cyclodextrins are commercially available products. The branched maltosyl cyclodextrin was prepared by the method described in U.S. Patent No. 4,668,626, supra.

Examples 1-12

The preparations in the following examples were prepared by mixing the indicated dose of human insulin with the selected saccharide or diluted saccharide (20 mg).

The preparations were tested in a rabbit model, where 4 to 8 rabbits were treated with the nasal insulin preparation (20 mg/animal). At 0, 15, 30, 60 and 120 min. after treatment blood samples were drawn and the concentration of blood glucose was determined. Each of these blood glucose concentrations were expressed as per cent of the initial glucose concentration, and by the triangle method the area between the average blood glucose vs. time curve and the curve f(time) = 100 was calculated. An index comparing the hypoglycaemic effect of the nasal preparation with that of a standard subcutaneous injection of a fast acting insulin preparation is then calculated according to the formula:

$$Index = 0.053 \times A/D$$

wherein A is the area over the curve for the test preparation, D is the dose of the test preparation in IU/animal, and the factor 0.053 is an empirically derived factor from a subcutaneous application of a fast acting insulin preparation.

| Example No. | Saccharide | Insulin dosage, IU | Index |
|---|---|---|---|
| 1 | D-mannose | 8 | 20 |
| 2 | L-sorbose | 8 | 21 |
| 3 | D-lyxose | 8 | 26 |
| 4 | D-xylose | 8 | 26 |
| 5 | D-ribose | 8 | 29 |
| 6 | D-ribose-5-phosphate | 6 | 30 |
| 7 | α-cyclodextrin | 6 | 42 |
| 8 | β-cyclodextrin | 6 | 44 |
| 9 | γ-cyclodextrin | 6 | 26 |
| 10 | Maltosyl cyclodextrin | 6 | 20 |
| 11 | β-cyclodextrin (80%) + Mg stearate (20%) | 6 | 38 |
| 12 | β-cyclodextrin (40%) + Mg stearate (60%) | 6 | 35 |

## Example 13

4.53 mg of human insulin were ground in a mortar with 395.47 mg of α-cyclodextrin to give a preparation with an insulin content of 0.3 IU/mg.

When tested in the rabbit model of Example 1 - 12, an index of 42 was found for this preparation.

## Example 14

2.91 mg of human insulin were ground in a mortar with 717.09 mg of α-cyclodextrin.

The ground powder was sprayed with a solution of 80 mg of didecanoyl phosphatidylcholine in an adequate volume of dilute ethanol containing about 30% (vol/vol) of water and was then allowed to dry.

The dry powder was passed through a 0.7 mm mesh sieve and finally through a 0.2 mm mesh sieve to give a preparation with an insulin content of 0.1 IU/mg.

When tested in the rabbit model of Example 1 - 12, an index of 88 was found for this preparation.

## Example 15

The procedure described in Example 14 was followed with 9.06 mg of human insulin, 750.94 mg of ribose and 40 mg of didecanoyl phosphatidylcholine to give a preparation with an insulin content of 0.3 IU/mg.

When tested in the rabbit model of Example 1 - 12, an index of 22 was found for this preparation.

## Example 16

The procedure described in Example 14 was followed with 2.3 mg of human insulin, 537.7 mg of α-cyclodextrin and 60 mg of didecanoyl phosphatidylcholine dissolved in ethanol containing about 4% (vol/vol) of water to give a preparation with an insulin content of 0.1 IU/mg.

When tested in the rabbit model of Example 1 - 12, an index of 95 was found for this preparation.

## Example 17

4 mg of human glucagon were ground in a mortar with 796 mg of α-cyclodextrin to give a preparation with a glucagon content of 5 mg/g.

The preparation was tested in a rabbit model, where 4 to 8 rabbits were treated with the nasal glucagon preparation (20 mg/animal). At 0, 15, 30, 60 and 120 min. after treatment, blood samples were drawn, and the concentration of blood glucose was determined. Each blood glucose concentration was expressed as per cent of the initial glucose concentration and by the triangle method the area between the curve t(time) = 100 and the average blood glucose vs. time curve was calculated. This area divided by the administered dose is used as a measure of the hyperglycaemic effect of the treatment.

In this test the present preparation gave an area per dose of 74,876.

## Example 18

The procedure described in Example 14 was followed with 4 mg of human insulin, 716 mg of α-cyclodextrin and 80 mg of didecanoyl phosphatidylcholine dissolved in ethanol containing about 4% (vol/vol) of water to give a preparation with a glucagon content of 5 mg/g.

When tested as described in example 17 this preparation gave an area per dose of 107,683.

The features disclosed in the foregoing description, in the following claims and/or in the accompanying drawings may, both separately and in any combination thereof, be material for realising the invention in diverse forms thereof.

## Claims

1. A preparation for non-enteral, trans-mucosal drug delivery characterized by comprising a pharmaceutically active agent and an absorption enhancing vehicle comprising at least one saccharide or a metabolite thereof, said saccharide being selected from the group consisting of a monosaccharide containing from 3 to 8 carbon atoms and an oligosaccharide containing from 6 to 16 monosaccharide containing from 3 to 8 carbon atoms, preferably glucosyl, residues.

2. A preparation according to Claim 1, wherein the monosaccharide simpliciter is selected from the group consisting of a tetrose, a pentose, a hexose and a heptose.

3. A preparation according to Claim 2, wherein the monosaccharide is D-erythrose, D-ribose, D-ribulose, D-xylose, D-lyxose, L-arabinose, D-mannose, L-sorbose or D-sedoheptulose.

4. A preparation according to Claim 3, wherein the monosaccharide is D-lyxose, D-xylose, D-ribose, D-mannose or L-sorbose.

5. A preparation according to Claim 1, wherein the oligosaccharide is selected from the group consisting of α-cyclodextrin, α-cyclodextrin, β-cyclodextrin and a branched cyclodextrin.

6. A preparation according to Claim 5, wherein the oligosaccharide is α- or β-cyclodextrin.

7. A preparation according to Claim 1-6, wherein the pharmaceutically active agent is a polypeptide.

8. A preparation according to Claim 7, wherein the polypeptide is insulin, an insulin derivative, a mixture of insulin and at least one insulin derivative, or a mixture of insulin derivatives.

9. A preparation according to Claim 8, wherein the insulin content is in the range of from 5 to 10000, preferably from 50 to 5000 international units per gram of the preparation.

10. A preparation according to Claim 7, wherein the polypeptide is glucagon.

11. A preparation according to Claim 10, wherein the glucagon content is in the range from 0.1 to 800, preferably from 1 to 600 milligrams per gram of the preparation.

12. A method of making a preparation for non-enteral, trans-mucosal drug delivery, which method comprises dispersing a pharmaceutically active agent in a vehicle comprising at least one saccharide as defined in Claim 1-6 and, optionally, one or more ancillary constituents selected from the group consisting of a powdery, waxy or liquid diluent, a pH-buffering agent, a preservative and an osmotic pressure controlling agent.

13. The use of a saccharide or metabolite thereof as defined in Claim 1 and a pharmaceutically active agent for the manufacture of a medicament for non-enteral, trans-mucosal drug delivery.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | PATENT ABSTRACTS OF JAPAN, vol. 8, no. 279 (C-257)[1716], 20th December 1984; & JP-A-59 148 717 (TAKEDA YAKUHIN KOGYO K.K.) 25-08-1984 * Abstract * | 1,5-11, 13 | A 61 K 47/00 A 61 K 37/24 |
| X | EP-A-0 192 263 (ISHIHARA SANGYO KAISHA LTD) * Page 2, lines 9-22; page 8, line 22; page 10, lines 13-22; claims 1,5 * | 1,5,6, 12,13 | |
| Y | DE-A-3 302 319 (THEURER et al.) * Claims 1,3; page 6, lines 17-30; example 3 * | 1-4,7,8 ,10 | |
| Y,D | EP-A-0 122 036 (TEIJIN LTD) * Page 3, lines 5-19; page 4, lines 34-38; page 5, lines 1-27; claims 1,12-14 * & US-A-4 613 500 | 1-4,7,8 ,10 | |
| A,D | EP-A-0 272 097 (NOVO INDUSTRI A/S) * Page 3, lines 10-34; page 4, lines 9-16; claims 10-15 * & DK-A- 37 00/87 | 1,5-10, 12,13 | TECHNICAL FIELDS SEARCHED (Int. Cl.4) A 61 K |

The present search report has been drawn up for all claims

| Place of search | Date of completion of the search | Examiner |
|---|---|---|
| THE HAGUE | 22-12-1988 | GAC G. |